# EUROPEAN PATENT SPECIFICATION

(11) **EP 1 949 868 B1**
(45) Date of publication and mention of the grant of the patent: **01.12.2010**
(21) Application number: 08150550.5
(22) Date of filing: 23.01.2008
(51) Int. Cl.: A61B 19/00

(54) **Washing device and method for medical instruments**
Waschvorrichtung und -verfahren für medizinische Instrumente
Dispositif et procédé de lavage pour instruments médicaux

(30) Priority: 26.01.2007 IT UD20070015
(43) Date of publication of application: 30.07.2008
(73) Proprietor: STEELCO SPA, 31039 Riese Pio X (IT)
(72) Inventor: Zardini, Fabio, 31033, Castelfranco Veneto (TV) (IT)
(74) Representative: Petraz, Gilberto Luigi

(56) References cited:
- EP-A- 1 398 004
- WO-A-01/91931
- WO-A-96/41686
- US-A- 5 313 934
- US-A1- 2005 000 553

## Description

### FIELD OF THE INVENTION

The present invention concerns a washing device for washing medical instruments of the tubular type made of various materials, in particular metal, plastic and/or rubber, used in medicine to explore, to examine by means of optical instruments or to empty natural cavities of the patient's body, and introduced through natural or artificial ways. Said washing medical instruments typically have a flexible tube, also called insertion tube, able to be introduced into the patient's body. In particular, said washing device can be used for the washing, chemical disinfection, drying and draining of one or more instruments of the endoscopic type, such as colonoscopes, bronchoscopes and gastroscopes.

### BACKGROUND OF THE INVENTION

It is known to wash medical instruments of the endoscopic type, which become dirty in use, in a suitable washing chamber of a washing machine. Endoscopic instruments typically have a flexible tube, also called insertion tube, able to be introduced into the patient's body, with a length, from tens of centimeters up to about 1,7 meter, and a diameter that varies according to the application, inside which channels of various dimensions and for various uses, for example biopsies, for an inspection using a video camera, for illumination, to spray air and/or water, are disposed or made. Inside the washing chamber, the complete washing treatment of both the external surfaces, the internal surfaces and the channels of the endoscopic instrument is effected, by means of washing liquids, such as water and detergent, in a first step, and by means of chemical disinfectant, for example based on glutaraldehyde or peracetic acid, in a second step.

The endoscopic instrument is washed inside the washing chamber of a suitable washing machine. Flexible tubes are provided inside the washing chamber in order to connect it to the internal channels of the endoscopic instrument that, by means of suitable water-tight connector, are located in connection with devices for the delivery of the washing liquid, associated with the washing machine.

The treatment of the external surface of the endoscopic instrument occurs, instead, by immersion in a container, for example an auxiliary tube, in the washing chamber of a suitable washing machine. The washing liquid is delivered into the auxiliary tube by means of a sleeve mounted thereon, so as to reach the entire external surface of the insertion tube.

As well as this, the auxiliary tube protects the insertion tube from unwanted knocks and rubbing during washing.

Furthermore, as the insertion tube is usually very long, in order to be easily disposed in the basket and loaded into the washing chamber, it has to be rolled in a spiral shape and kept in such spiral shape in the washing chamber. For this purpose, normally, the auxiliary tube is spiral shaped.

However, this type of treatment has the disadvantage that, as the insertion tube has to be inserted into the auxiliary tube, usually spiral shaped, contact points between these two tubes are formed which, inevitably, determine some zones, on the external surface of the endoscopic instrument, where the treatment is not performed or is performed only partially, forming so-called "shadow zones" of the treatment, considerably reducing the effectiveness of the overall washing treatment.

EP-A-1.398.004 discloses a cleaning device which is mounted in a surgical treatment instrument so as to be attachable/detachable and in which a cleaning cover is disposed and includes a jet port opening toward the distal-end function portion of the surgical treatment instrument and which supplies cleaning water into the cleaning cover via the jet port to clean only the distal-end function portion disposed in the cleaning water.

The disclosed cleaning device is suitable only for "short" distal-end function portion of surgical treatment device, that are rigid and that can be easily handled but it is not suitable for very long insertion tube of the type as above-discussed, usually of the flexible type too, because of the increased resulting overall dimensions.

WO-A-96/41686 discloses a device for sterilization of a dental instrument comprising a chamber with two walls separated by a hollow space, subdivided along axis into smaller annular spaces, separated each other and connected to the interior of the chamber by means of rings of holes with downward pointing jets. These jets sterilize the internal and external surface area of the instrument along its length.

Also the sterilizing device disclosed in WO'686 is unsuitable for cleaning very long insertion tube of the type as above-discussed, usually of the flexible type too, because it would result in increased resulting overall dimensions, with a very long sequence of separated annular spaces, each provided with its own valve and fed with a separated portion of the main flow.

Purpose of the present invention is to achieve a washing device for washing medical instruments, in particular tubular instruments of the endoscopic type, which allows an efficient and uniform cleaning of the endoscopic instrument and guarantees the repeatability of the cleaning operation of the instrument inside predetermined standards of hygiene.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, a washing device for washing a medical instrument of the tubular type, typically provided with an insertion tube of the flexible type, comprises at least a first tubular element and delivery means able to deliver a washing liquid inside the first tubular element, towards the medical instrument.

The washing device further comprises a second tubular element inside which the medical instrument is able to be housed and which is disposed inside the first tubular element, coaxially thereto, so as to define an interspace between the tubular elements, in which said washing liquid is able to flow. Furthermore, the second tubular element is provided with a plurality of fissures that put the inside of the second tubular element in communication with the interspace, so that the washing liquid is able to pass from the interspace towards the medical instrument.

In accordance with a characteristic feature of the present invention, said first tubular element and said second tubular element are curvilinear, in order to adapt to the shape of the aforementioned medical instrument, and the fissures are disposed substantially along the entire length of the second tubular elements.

The curvilinear shape of the first and second tubular element is provided in order to minimize the overall dimensions of the washing device, in respect to a hypothetic linear configuration. This enables the flexible medical instrument to assume a space-saving configuration curved along the length of the first and second tubular elements when housed into the washing device according to the invention, and permits, at the same time, an efficient washing operation of the medical instrument. The first and the second tubular elements can have a development of from tens of centimeters to about 2 meter. This is particularly advantageous when the insertion tube of the medical instrument reaches a length up to about 1,7 meter.

An advantageous form of embodiment of the present invention provides to achieve the two tubular elements in a spiral shape, so improving said space-saving effect. To this end, the spiral shape can be provided with a linear development of up to about 2 meter.

According to a further advantageous form of embodiment of the present invention, said interspace is a single interspace between said first and second tubular elements that develops substantially along the entire length of said tubular elements, in order to deviate the flow of the washing liquid from an axial flow along the interspace in a radial flow through the second tubular element, thus determining an homogeneous and uniform cleaning effect on the whole surface of the medical instrument.

The present invention allows an effective and uniform cleaning of the medical instrument inserted therein and guarantees the repeatability of the cleaning operation of the instrument within predetermined standards of hygiene.

In particular, the present invention prevents the formation of "shadow zones" of the washing treatment, since, during the washing treatment, the medical instrument is maintained centered and distanced, with respect to the internal surface of the second tubular element, from the washing liquid that flows with a certain pressure.

In other words, the medical instrument is maintained distanced from the internal surface of the second tubular element by spacer means that consists of the same washing liquid as it flows.

Furthermore, the present invention prevents, or in any case considerably reduces, the wear and accidental breakages of the medical instrument subjected to washing, thus increasing the operative life thereof, with evident economic advantages.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a longitudinal section of a washing device according to the present invention found associated with a medical instrument to be washed;
- fig. 2 is a cross section of the washing device in fig. 1 associated with a medical instrument to be washed; and
- fig. 3 is perspective view of a washing device according to the present invention associated with a trolley of a washing machine.

### DETAILED DESCRIPTION OF A PREFERENTIAL FORM OF EMBODIMENT

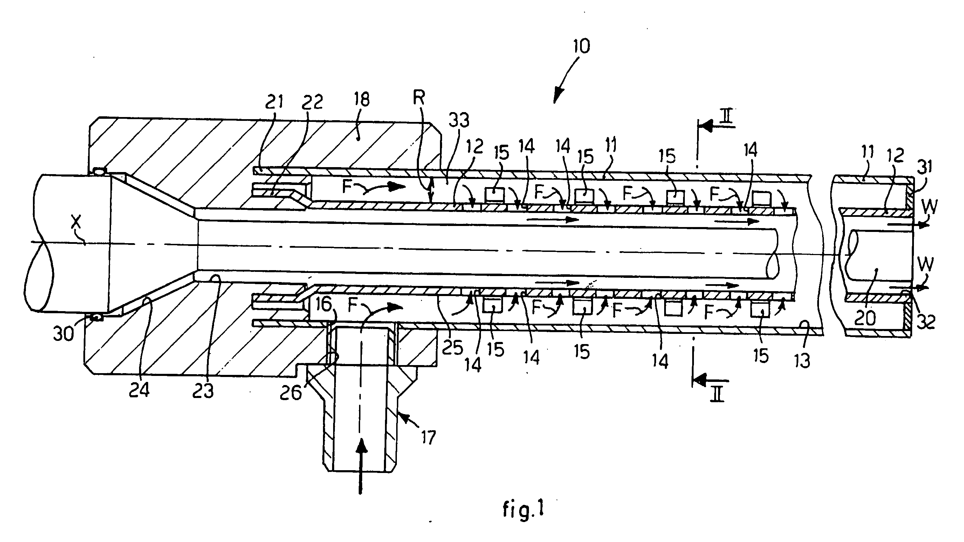

With reference to fig. 1, a washing device 10 according to the present invention can be used to wash an endoscopic instrument, provided with a plurality of internal channels, not shown in the drawings, and with an insertion tube, or catheter, 20, of the flexible type, for example with a length of about 1,7 meter.

The washing device 10 comprises an external tube 11 preferably with a circular section and with a longitudinal axis X, curved and wound in a spiral. The tube 11 is provided with a through hole 13, circular in shape, aligned with the longitudinal axis X, in which an internal tube 12 is disposed, also wound in a spiral and with a preferably circular section. The spiral has, for example, a development of about 2 meter, in order to meet all the necessities. The tube 12 therefore has an external diameter of less than the section of the hole 13 and is provided with a through hole 19, coaxial to the hole 13 and coaxial to the tube 11.

The catheter 20 is able to be housed in the through hole 19 of the tube 12, for the washing treatment. The flexible catheter 20, thus, adapts to the spiral shape of the washing device 10, when inserted into the tube 12, so minimizing the overall dimensions of the frame of the washing device 10 during the washing operation.

The tubes 11 and 12 can be made of flexible material such as a polymeric material, such as plastic or rubber, or a metal material.

Alternatively, the tubes 11 and 12 can be made of rigid material.

By construction, therefore, an annular interspace 33 is defined between the tubes 11 and 12, in which a washing liquid is able to flow for washing the catheter 20.

Fissures 14 are made radially on the lateral surface 25 of the internal tube 12 disposed substantially along the entire length of the tube 12, which put the interspace 33 and the hole 19 of the tube 12 in hydraulic communication. By means of the fissures 14 the washing liquid is able to pass from the interspace 33 towards the hole 19, inside the internal tube 12, so as to wash the catheter 20.

The fissures 14 are preferably made in a uniform manner and with a constant and predetermined pitch, both along the longitudinal axis X, and along the circular section of the internal tube 12, in order to obtain a uniform delivery of the washing liquid onto the catheter 20.

The disposition of the fissures 14 allows to generate a flow of the washing liquid that enters substantially in a radial direction towards the centre of the hole 19, hitting the catheter 20 uniformly, then moving axially along the tube 12 and draining outside (fig. 1).

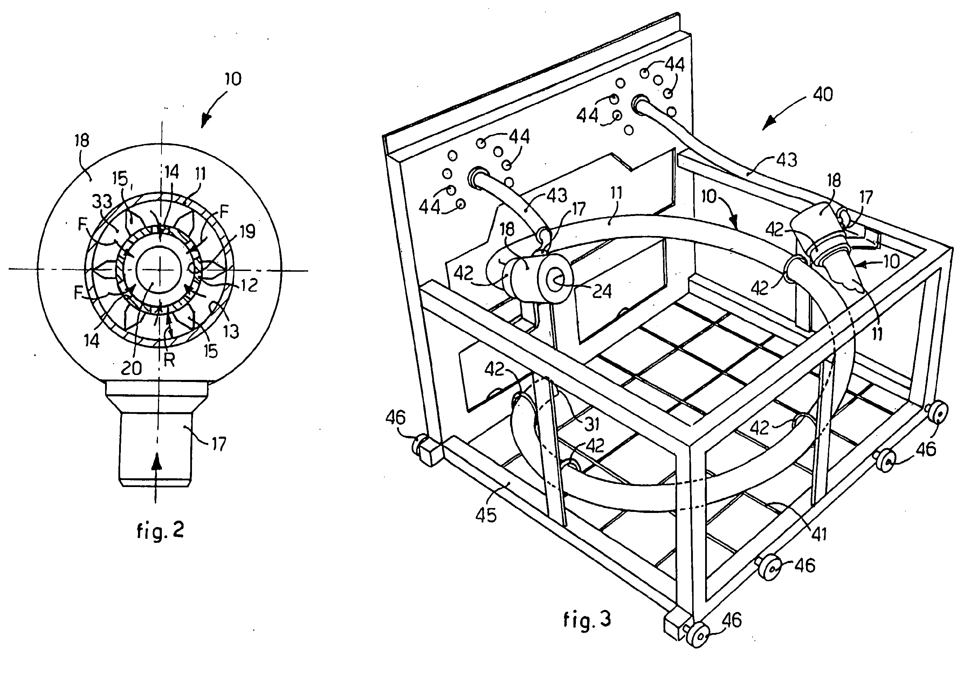

Thanks to the particular geometry with which the fissures 14 are made and disposed on the surface 25, the flow of washing liquid, indicated by the arrows F in figs. 1 and 2, which is directed with a particular delivery pressure, determines, in the hole 19 of the internal tube 12, a liquid bearing that maintains the catheter 20 centered in the internal tube 12, thus guaranteeing the effectiveness and the uniformity of the washing, eliminating possible shadow zones, that is, portions of catheter 20 not subjected to washing, and preventing undesired movements of the catheter 20.

In other words, the washing liquid works, not only as a cleaning means, but also as a distancing and centering means in order to maintain the catheter 20 centered and distanced from the internal surface of the tube 12 and to prevent the formation of shadow zones of the washing treatment.

In accordance with an advantageous form of embodiment of the present invention, the device 10 is provided with spacer elements 15 disposed inside the external tube 11, in the interspace 33, so as to maintain a predetermined distance R (figs. 1 and 2) between the tubes 11 and 12, which is also the height, in a radial direction of the interspace 33, in this specific case constant in the direction of the axis X, along said interspace 33. This is advantageous for preventing undesired movements both of the internal tube 12, in order to always maintain constant the section of the interspace 33 and prevent the formation of obstacles to the flow of the washing liquid, and also of the catheter 20, especially while it undergoes washing with the flow of liquid, so as to prevent any wear thereof.
Advantageously, the spacer elements 15 are made in a single piece with the internal tube 12, for example as teeth that protrude radially from the external lateral surface 36 of the tube 12. In this specific case, the spacer elements 15 are disposed alternating with the fissures 14 (figs. 1 and 2).
The spacer elements 15 also act, advantageously, as retaining elements in order to hold the tube 12 inside the tube 11.

The external tube 11 is provided with an opening 16, made on the lateral surface thereof, which puts the interspace 33 in communication with the exterior and which is associated with a sleeve 17 for the delivery of the washing liquid into the interspace 33 (figs. 1, 2 and 3).

The tubes 11 and 12 are also provided with respective front entrances 21, 22, which put the respective holes 13 and 19 in communication with the exterior, adjacent to each other and aligned with the longitudinal axis X, through which the catheter 20 is able to be inserted into the tubes 11 and 12.

Furthermore, the external tube 11 has a closing wall 31, disposed in a position opposite to the entrance 21, so as to close the interspace 33, while the internal tube 12 has an exit 32, which acts as a draining hole in order to discharge the washing liquid, as indicated by arrow W in fig. 1, in an opposite position to the entrance 22 thereof.

A coupling mouth 18, substantially cylindrical in shape, is mounted in correspondence with the entrance 21, and is therefore partially superimposed above the external 11 and the internal 12 tube, so as to favor the insertion of the catheter 20 (figs. 1 and 2).

In particular, the coupling mouth 18 has a passage 23 through which the catheter 20 can be inserted into the through hole 19 of the internal tube 12.

The passage 23 of the coupling mouth 18 has a part shaped as a lead-in, so as to facilitate the insertion of the catheter 20, and provided with a washer 30 that achieves the watertight seal between the catheter 20 and the coupling mouth 18.

The coupling mouth 18 is also provided with a lateral passage 26 which is disposed in correspondence with the opening 16 and is coupled with the sleeve 17, for the delivery of the washing liquid into the hole 13.

The device 10 can be used, for example, on a trolley 40 of the known type, shown in fig. 3, which is able to be inserted into a washing chamber of a washing machine for endoscopic instruments, not shown in the drawings.

The trolley 40 can be used to wash two endoscopic instruments simultaneously, using two devices 10, of which one is shown entirely and the other only partially in fig. 3.

The trolley 40 comprises a frame 45 which can run on rollers 46 and which supports a grid-like surface 41 on which the two devices 10 are disposed and supported by rings 42. The trolley 40 is provided with conduits 43 connecting it with the sleeve 17, for washing the external surface of each catheter 20, and a plurality of little tubes, the hydraulic/pneumatic intakes 44 of which are shown in fig. 3, which are able to be connected to each of the internal channels of the two endoscopic instruments, in order to wash them. Both the conduits 43, and the little tubes and the relative hydraulic/pneumatic intakes 44, are also able to be connected to respective pumps and selective delivery circuits of the washing liquid, disinfectant and air for drying, not shown in the drawings.

It is clear that modifications and/or additions of parts may be made to the washing device 10 as described heretofore, without departing from the scope of the present invention.

For example, the internal tube 12 can consist of a netted tubular element, with a mesh that is more or less wide, according to needs.

It is also clear that, although the present invention has been described with reference to some specific examples, a person of skill in the art shall certainly be able to achieve many other equivalent forms of washing device, having the characteristics as set forth in the claims and hence all coming inside the scope of protection defined thereby.

## Claims

1. Washing device (10) for washing a medical instrument (20) of the tubular type comprising at least a first tubular element (11) having a longitudinal axis (X), delivery means (17) able to deliver a washing liquid inside said first tubular element (11), towards said medical instrument (20), and a second tubular element (12) which is disposed inside said first tubular element (11), coaxial thereto, so as to define an interspace between said tubular elements (11, 12), in which said washing liquid is able to flow, wherein said medical instrument (20) is able to be housed inside said second tubular element (12), and wherein said second tubular element (12) is provided with a plurality of fissures (14) made radially on said second tubular element (12), which put the inside of said second tubular element (12) in communication with said interspace, so that said washing liquid is able to pass from said interspace towards said medical instrument (20), **characterized in that** said first tubular element (11) and said second tubular element (12) are curvilinear and spiral shaped, **in that** said interspace is a single interspace (33) that develops substantially along the entire length of said tubular elements (11, 12) and **in that** said radial fissures (14) are disposed substantially along the entire length of the second tubular element (12) with a constant and predetermined pitch, both along the longitudinal axis (X) and along the circular section of the second tubular element (12), so as to generate a flow of the washing liquid that enters substantially in a radial direction towards the centre of the second tubular element (12), hitting the medical instrument (20) uniformly so as to maintain the medical instrument (20) centered in the second tubular element (12), thus guaranteeing the effectiveness and the uniformity of the washing and eliminating possible shadow zone, then moving axially along the second tubular element (12) and draining outside.

2. Device as in claim 1, **characterized in that** said fissures (14) are made radially on the lateral surface (25) of said second tubular element (12).

3. Device as in any claim hereinbefore, **characterized in that** it comprises spacer elements (15) disposed in said interspace (33), between said first tubular element (11) and said second tubular element (12), and able to maintain a predetermined distance (R) between said first tubular element (11) and said second tubular element (12).

4. Device as in any claim hereinbefore, **characterized in that** said first tubular element (11) and said second tubular element (12) are made by means of a flexible material.

5. Device as in any claim from 1 to 3, **characterized in that** said first tubular element (11) and said second tubular element (12) are made by means of a rigid material.

6. Device as in any claim hereinbefore suitable for mounting on a trolley (40) able to be inserted into a washing chamber of a washing machine for endoscopic instruments and comprising a frame (45) which supports a grid-like surface (41) on which the devices (10) are able to be disposed and supported by rings (42), said trolley (40) being provided with conduits (43) connecting it with said delivery means (17) of said device, for washing the external surface of the medical instrument (20), and a plurality of hydraulic/pneumatic intakes (44) which are able to be connected to each of the internal channels of the medical instrument (20), in order to wash it.

7. Washing machine for endoscopic instruments comprising a device (10) as in any claim herein before and a washing chamber provided with a trolley (40) comprising a frame (45) which supports a grid-like surface (41) on which the device is able to be disposed and supported by rings (42), said trolley (40) being provided with conduits (43) connecting it with said delivery means (17), for washing the external surface of the medical instrument (20), and a plurality of hydraulic/pneumatic intakes (44) which are able to be connected to each of the internal channels of the medical instrument (20), in order to wash it.

8. Method for washing a medical instrument (20) of the tubular type in a device (10) provided with a tubular element (12) in which said medical instrument (20) is able to be housed, **characterized in that**, in a washing step, said medical instrument (20) is maintained distanced from the internal surface of said tubular element (12) by means of spacer means and is washed by means of a washing liquid that flows inside said tubular element (12), wherein said spacer means is formed by the same washing liquid as it flows and wherein the flow of fluid is firstly axially directed along a single annular interspace (33) that develops along a curvilinear path between the tubular element (12) itself and an external tubular element (11) and then it is radially deviated toward the medical instrument (20), so that the washing liquid passes from said interspace (33) towards said medical instrument (20), hitting the medical instrument (20) uniformly so as to maintain the medical instrument (20) centered in the second tubular element (12), thus guaranteeing the effectiveness and the uniformity of the washing and eliminating possible shadow zone, then moving axially along the second tubular element (12) and draining outside.

9. Method as in claim 8, **characterized in that** said second tubular element (12) is provided with a plurality of fissures (14) made radially on said second tubular element (12), said radial fissures (14) being disposed substantially along the entire length of the second tubular elements (12) with a constant and predetermined pitch, both along the longitudinal axis (X) of the external tubular element (11) and along the circular section of the tubular element (12), thus determining a homogeneous and uniform cleaning effect on the whole surface of the medical instrument (20).

## Patentansprüche

1. Waschvorrichtung (10) zum Waschen eines rohrförmigen medizinischen Instruments (20), mit mindestens einem eine Längsachse (X) aufweisenden rohrförmigen Element (11), einer Zuführeinrichtung (17), die eine Waschflüssigkeit innerhalb des ersten rohrförmigen Elements (11) dem medizinischen Instrument (20) zuführen kann, und einem innerhalb des ersten rohrförmigen Elements (11) koaxial dazu so angeordneten zweiten rohrförmigen Element (12), dass ein Zwischenraum zwischen den rohrförmigen Elementen (11, 12) definiert ist, in dem die Waschflüssigkeit strömen kann, wobei das medizinische Instrument (20) innerhalb des zweiten rohrförmigen Elements (12) aufgenommen werden kann und das zweite rohrförmige Element (12) mit mehreren radial ausgebildeten Spalten (14) versehen ist, die das Innere des zweiten rohrförmigen Elements (12) mit dem Zwischenraum verbinden, so dass die Waschflüssigkeit vom Zwischenraum zum medizinischen Instrument (20) gelangen kann, **dadurch gekennzeichnet, dass** das erste rohrförmige Element (11) und das zweite rohrförmige Element (12) krummlinig und spiralförmig geformt sind, dass der Zwischenraum ein im Wesentlichen entlang der Gesamtlänge der rohrförmigen Elemente (11, 12) ausgebildeter einzelner Zwischenraum (33) ist und dass die Radialspalte (14) im Wesentlichen entlang der Gesamtlänge des zweiten rohrförmigen Elements (12), sowohl entlang der Längsachse (X) als auch entlang des kreisförmigen Querschnitts des zweiten rohrförmigen Elements (12), mit konstanter und vorbestimmter Teilung angeordnet sind, um eine im Wesentlichen in Radialrichtung zur Mitte des zweiten rohrförmigen Elements (12) eintretende Strömung der Wasarflüssigkeit zu erzeugen, die auf das medizinische Instrument (20) gleichförmig auftrifft, um es mittig im zweiten rohrförmigen Element (12) zu halten, wobei dadurch die Effektivität und die Gleichförmigkeit des Waschens und des Eliminierens möglicher Schattenbereiche garantiert wird, die danach axial entlang des zweiten rohrförmigen Elements (12) fortschreitet und nach außen abläuft.

2. Vorrichtung nach Anspruch 1, **dadurch gekennzeichnet, dass** die Spalte (14) radial an der Seitenfläche (25) des zweiten rohrförmigen Elements (12) ausgebildet sind.

3. Vorrichtung nach einem der vorhergehenden Ansprüche, **dadurch gekennzeichnet, dass** sie Abstandselemente (15) enthält, die in dem Zwischenraum (33) zwischen dem ersten rohrförmigen Element (11) und dem zweiten rohrförmigen Element (12) angebracht sind und einen vorgegebenen Abstand (R) zwischen dem ersten rohrförmigen Element (11) und dem zweiten rohrförmigen Element (12) erhalten können.

4. Vorrichtung nach einem der vorhergehenden Ansprüche, **dadurch gekennzeichnet, dass** das erste rohrförmige Element (11) und das zweite rohrförmige Element (12) aus einem flexiblen Material bestehen.

5. Vorrichtung nach einem der Ansprüche 1 bis 3, **dadurch gekennzeichnet, dass** das erste rohrförmige Element (11) und das zweite rohrförmige Element (12) aus einem starren Material bestehen.

6. Vorrichtung nach einem der vorhergehenden Ansprüche, geeignet zum Montieren auf einen Rollwagen (40), der in eine Waschkammer einer Waschmaschine für endoskopische Instrumente eingeführt werden kann und ein Gerüst (45) enthält, das eine gitterartige Fläche (41) trägt, auf dem die Vorrichtungen (10) durch Ringe (42) angeordnet und getragen werden können, wobei der Rollwagen (40) mit Leitungen (43), die ihn mit der Zuführeinrichtung (17) der Vorrichtung zum Waschen der Außenflächen des medizinischen Instruments (20) verbinden, und mit mehreren hydraulisch/pneumatischen Einlässen (44) versehen ist, die mit jedem der internen Kanäle des medizinischen Instruments (20) verbunden werden können, um es zu waschen.

7. Waschmaschine für endoskopische Instrumente mit einer Vorrichtung (10) nach einem der vorhergehenden Ansprüche und einer Waschkammer, die mit einem Rollwagen (40) versehen ist, der ein eine gitterartige Fläche (41) tragendes Gerüst (45) enthält, auf dem die Vorrichtung durch Ringe (42) angeordnet und getragen werden kann, wobei der Rollwagen (40) mit Leitungen (43), die ihn mit der Zuführeinrichtung (17) zum Waschen der Außenfläche des medizinischen Instruments (20) verbinden, und mit mehreren hydraulisch/pneumatischen Einlässen (44) versehen ist, die mit jedem der internen Kanäle des medizinischen Instruments (20) verbindbar sind, um es zu waschen.

8. Verfahren zum Waschen eines rohrförmigen medizinischen Instruments (20) in einer Vorrichtung (10), die mit einem rohrförmigen Element (12) versehen ist, in dem das medizinische Instrument (20) aufgenommen werden kann, **dadurch gekennzeichnet, dass** beim Waschen das medizinische Instrument (20) mittels einer Abstandseinrichtung auf Abstand von der Innenfläche des rohrförmigen Elements (12) gehalten wird und mittels einer innerhalb des rohrförmigen Elements (12) strömenden Waschflüssigkeit gewaschen wird, wobei die Abstandseinrichtung durch dieselbe strömende Waschflüssigkeit gebildet ist und wobei die Fluidströmung zuerst axial entlang eines einzelnen ringförmigen Zwischenraums (33) gerichtet ist, der sich entlang eines krummlinigen Pfades zwischen dem rohrförmigen Element (12) selbst und einem externen rohrförmigen Element (11) ausbildet, und danach radial zum medizinischen Instrument (20) hin abgelenkt wird, so dass die Waschflüssigkeit vom Zwischenraum (33) zum medizinischen Instruments (20) gelangt, auf das medizinische Instrument (20) gleichförmig auftrifft, um es im zweiten rohrförmigen Element (12) mittig zu halten, womit die Effektivität und die Gleichheit des Waschens und des Eliminierens möglicher Schattenbereiche garantiert wird, danach axial entlang des zweiten rohrförmigen Elements (12) fortschreitet und nach außen abläuft.

9. Verfahren nach Anspruch 8, **dadurch gekennzeichnet, dass** das zweite rohrförmige Element (12) mit mehreren radial angebrachten Spalten (14) versehen ist, wobei die Radialspalte (14) im Wesentlichen entlang der Gesamtlänge des zweiten rohrförmigen Elements (12), sowohl entlang der Längsachse (X) des externen rohrförmigen Elements (11) als auch entlang des kreisförmigen Querschnitts des rohrförmigen Elements (12), mit einem konstanten und vorgegebenen Abstand angebracht sind, wodurch ein homogener und gleichmäßiger Säuberungseffekt an der ganzen Oberfläche des medizinischen Instruments (20) erreicht wird.

## Revendications

1. Dispositif de lavage (10) pour laver un instrument médical (20) du type tubulaire comprenant au moins un premier élément tubulaire (11) possédant un axe longitudinal (X), des moyens de distribution (17) capables de distribuer un liquide de lavage à l'intérieur dudit premier élément tubulaire (11), vers ledit instrument médical (20), et un second élément tubulaire (12) qui est disposé à l'intérieur dudit premier élément tubulaire (11), coaxial par rapport à celui-ci, afin de définir un espace intermédiaire entre lesdits éléments tubulaires (11, 12), dans lequel ledit liquide de lavage est capable de s'écouler, dans lequel ledit instrument médical (20) est capable d'être logé à l'intérieur dudit second élément tubulaire (12), et dans lequel ledit second élément tubulaire (12) est pourvu d'une pluralité de fissures (14) réalisées de façon radiale sur ledit second élément tubulaire (12), qui mettent l'intérieur dudit second élément tubulaire (12) en communication avec ledit espace intermédiaire, de sorte que ledit liquide de lavage soit capable de passer à partir dudit espace intermédiaire vers ledit instrument médical (20), **caractérisé en ce que** ledit premier élément tubulaire (11) et ledit second élément tubulaire (12) présentent des formes curviligne et en spirale, **en ce que** ledit espace intermédiaire est un espace intermédiaire unique (33) qui se développe sensiblement sur la longueur entière desdits éléments tubulaires (11, 12) et **en ce que** lesdites fissures radiales (14) sont disposées sensiblement sur la longueur entière du second élément tubulaire (12) avec un pas constant et prédéterminé, à la fois le long de l'axe longitudinal (X) et le long de la section circulaire du second élément tubulaire (12), afin de produire un écoulement du liquide de lavage qui entre sensiblement dans une direction radiale vers le centre du second élément tubulaire (12), entrant en contact avec l'instrument médical (20) uniformément afin de maintenir l'instrument médical (20) centré dans le second élément tubulaire (12), garantissant ainsi l'efficacité et l'uniformité du lavage et éliminant une possible zone d'ombre, puis se déplaçant de façon axiale le long du second élément tubulaire (12) et étant évacué vers l'extérieur.

2. Dispositif selon la revendication 1, **caractérisé en ce que** lesdites fissures (14) sont réalisées de façon radiale sur la surface latérale (25) dudit second élément tubulaire (12).

3. Dispositif selon une quelconque revendication précédente, **caractérisé en ce qu'**il comprend des éléments entretoises (15) disposés dans ledit espace intermédiaire (33), entre ledit premier élément tubulaire (11) et ledit second élément tubulaire (12), et capables de maintenir une distance prédéterminée (R) entre ledit premier élément tubulaire (11) et ledit second élément tubulaire (12).

4. Dispositif selon une quelconque revendication précédente, **caractérisé en ce que** ledit premier élément tubulaire (11) et ledit second élément tubulaire (12) sont réalisés au moyen d'un matériau flexible.

5. Dispositif selon une quelconque des revendications 1 à 3, **caractérisé en ce que** ledit premier élément tubulaire (11) et ledit second élément tubulaire (12) sont réalisés au moyen d'un matériau rigide.

6. Dispositif selon une quelconque revendication précédente approprié pour être monté sur un chariot (40) capable d'être inséré dans une chambre de lavage d'une machine de lavage pour des instruments endoscopiques et comprenant un cadre (45) qui supporte une surface en forme de grille (41) sur laquelle les dispositifs (10) sont capables d'être disposés et supportés par des bagues (42), ledit chariot (40) étant pourvu de conduits (43) le raccordant avec lesdits moyens de distribution (17) dudit dispositif, pour laver la surface externe de l'instrument médical (20), et une pluralité d'admissions hydrauliques/pneumatiques (44) qui sont capables d'être raccordées à chacun des canaux internes de l'instrument médical (20), afin de le laver.

7. Machine de lavage pour des instruments endoscopiques comprenant un dispositif (10) selon une quelconque revendication précédente et une chambre de lavage pourvue d'un chariot (40) comprenant un cadre (45) qui supporte une surface en forme de grille (41) sur laquelle le dispositif est capable d'être disposé et supporté par des bagues (42), ledit chariot (40) étant pourvu de conduits (43) le raccordant avec lesdits moyens de distribution (17), pour laver la surface externe de l'instrument médical (20), et une pluralité d'admissions hydrauliques/pneumatiques (44) qui sont capables d'être raccordées à chacun des canaux internes de l'instrument médical (20), afin de le laver.

8. Procédé pour laver un instrument médical (20) du type tubulaire dans un dispositif (10) pourvu d'un élément tubulaire (12) dans lequel ledit instrument médical (20) est capable d'être logé, **caractérisé en ce que**, dans une étape de lavage, ledit instrument médical (20) est maintenu de façon éloigné de la surface interne dudit élément tubulaire (12) grâce à des moyens entretoises et est lavé au moyen d'un liquide de lavage qui s'écoule à l'intérieur dudit élément tubulaire (12), dans lequel lesdits moyens entretoises sont formés par le même liquide de lavage lorsqu'il s'écoule et dans lequel l'écoulement de fluide est d'abord dirigé de façon axiale le long d'un espace intermédiaire annulaire unique (33) qui se développe le long d'un trajet curviligne entre l'élément tubulaire (12) lui-même et un élément tubulaire externe (11) et est ensuite dévié de façon radiale vers l'instrument médical (20), de sorte que le liquide de lavage passe à partir dudit espace intermédiaire (33) vers ledit instrument médical (20), entrant en contact avec l'instrument médical (20) uniformément afin de maintenir l'instrument médical (20) centré dans le second élément tubulaire (12), garantissant ainsi l'efficacité et l'uniformité du lavage et éliminant une possible zone d'ombre, puis se déplaçant de façon axiale le long du second élément tubulaire (12) et étant évacué vers l'extérieur.

9. Procédé selon la revendication 8, **caractérisé en ce que** ledit second élément tubulaire (12) est pourvu d'une pluralité de fissures (14) réalisées de façon radiale sur ledit second élément tubulaire (12), lesdites fissures radiales (14) étant disposées sensiblement sur la longueur entière du second élément tubulaire (12) avec un pas constant et prédéterminé, à la fois le long de l'axe longitudinal (X) de l'élément tubulaire externe (11) et le long de la section circulaire de l'élément tubulaire (12), déterminant ainsi un effet de nettoyage homogène et uniforme sur la surface entière de l'instrument médical (20).
